# EUROPÄISCHE PATENTANMELDUNG

(11) **EP 1 338 260 A1**
(43) Veröffentlichungstag der Anmeldung: **27.08.2003**
(21) Anmeldenummer: 03003100.9
(22) Anmeldetag: 13.02.2003
(51) Int. Cl.: A61F 13/00, A61F 13/02, A61F 13/06, A61F 13/10

(54) **Kompressions- und Stützbandage**

(30) Priorität: 21.02.2002 CH 3072002; 08.11.2002 CH 18812002
(71) Anmelder: Saupe AG, 9302 Kronbühl (CH)
(72) Erfinder: Saupe, Rolf, 9302 Kronbühl (CH)
(74) Vertreter: Römpler, Aldo

(57) **Zusammenfassung**

Die Kompressions- und Stützbandage (12) ist an ihrer dem Körper zuzuwendenden Bandagenfläche (4) mit einem rutschhemmenden Bereich (3) versehen. Dieser ist vorzugsweise als Streifen ausgebildet, der entlang jeweils einer der beiden Längskanten (7, 8) verläuft. Denkbar sind indessen auch zwei oder mehr parallel zueinander liegende Streifen, die zusätzlich auch an der vom Körper abzuwendenden Bandagenfläche (10) angeordnet sein können. Der rutschhemmende Bereich (3, 6) besteht beispielsweise aus Silikon. Dieses kann als dünne Schicht auf das Gewebe der Kompressions- und Stützbandage (12) aufgebracht werden. Dies wirkt beim Tragen am Körper stark rutschhemmend, wird aber nicht als störend empfunden. Weder beim Gehen noch bei körperlicher Betätigung, kann die Kompressions- und Stützbandage (12) wegrutschen oder rümpfen. Ein Ende der Kompressions- und Stützbandage weist vorteilhaft eine Tasche zur Aufnahme von Befestigungsmitteln, z.B. Klammern. Dadurch sind diese beim Waschen oder Nichtgebrauch der Kompressions- und Stützbandage geschützt untergebracht.

## Beschreibung

Die vorliegende Erfindung betrifft eine Kompressions- und Stützbandage.

Die bekannten Kompressions- und Stützbandagen bestehen gewöhnlich aus einem Gewebe. Es gibt sie als Wickelbandage, d.h. als zu wickelndes Band, oder schlauchförmig, zum Überziehen. Häufig werden diese Bandagen am Bein verwendet. Hier können sie verschiedene Funktionen erfüllen, beispielsweise bei Krampfadern. Besonders gelangen sie aber unterstützend und schonend bei Schädigung von Muskeln und/oder Gelenken zur Anwendung. Hierzu werden sie am oder oberhalb des Knies getragen. Oft ist dies auch beim Sport der Fall. Die Kompressions- und Stützbandage ist in jedem Fall längere Zeit zu tragen. Deren Funktion wird indessen nur dann erfüllt, wenn die Kompressions- und Stützbandage in der ihr zugedachten Lage bleibt. Schon beim normalen Gehen, erst recht aber bei körperlicher Betätigung, rutscht die Kompressions- und Stützbandage weg und/oder rümpft. Dabei wird nicht nur der Zweck der Bandage nicht mehr zuverlässig erfüllt, sondern es bilden sich auch störende Druckstellen. Diese Nachteile lassen sich auch durch ein besonders straffes Umwickeln oder durch die Wahl einer besonders engen und straff sitzenden schlauchförmigen Kompressions- und Stützbandage nicht vermeiden. Dadurch wird das Tragen nur von vornherein derart unangenehm, dass die Bandage bereits nach kurzer Zeit wieder abgenommen werden muss. Folglich wird oft auf deren Hilfe verzichtet und beispielsweise ein Fortschreiten oder ein langwierigeres Abheilen der Schädigung von Muskeln und/oder Gelenken in Kauf genommen.

Auf der Grundlage dieser Erkenntnisse setzt sich die Erfindung die Aufgabe, eine Kompressions- und Stützbandage zu schaffen, die auch nach längerem Tragen in der ihr zugedachten Lage bleibt, ohne dabei zu stören. Die Kompressions- und Stützbandage soll zudem problemlos gewaschen und bei Nichtgebrauch aufgehoben werden können.

Die erfindungsgemässe Kompressions- und Stützbandage entspricht den kennzeichnenden Merkmalen des Patentanspruchs 1. Weitere vorteilhafte Ausbildungen des Erfindungsgedankens sind aus den abhängigen Patentansprüchen ersichtlich.

Nachfolgend werden bevorzugte Ausführungsbeispiele der Erfindung anhand der Zeichnung näher beschrieben.
- Fig. 1: zeigt eine schlauchförmige Kompressions- und Stützbandage;
- Fig. 2 - 4: zeigen jeweils in schematischer Detailvergrösserung drei Ausführungsbeispiele der Kompressions- und Stützbandage;
- Fig. 5: zeigt ein Beispiel einer bandförmige, zu wickelnden Kompressions- und Stützbandage; und
- Fig. 6: zeigt ein weiteres Beispiel einer Bandage nach Fig. 5;
- Fig. 7 - 8: zeigen ein Beispiel einer Bandage mit einer Aufnahme für Befestigungsmittel.

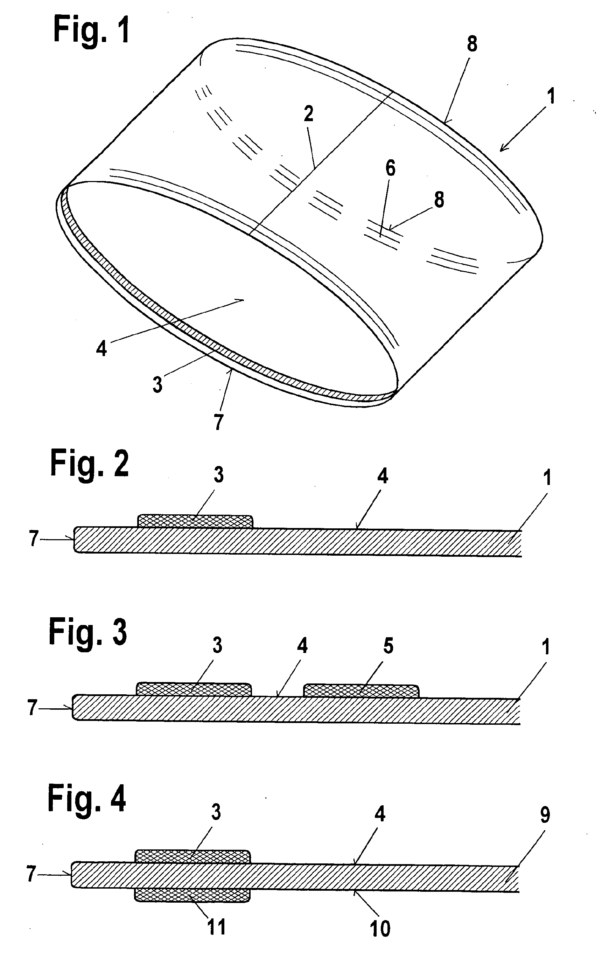

Die schlauchförmige Kompressions- und Stützbandage 1 nach Fig. 1 ist dazu bestimmt über den zu schützenden Körperteil gezogen zu werden, beispielsweise über ein Bein. Sie kann in verschiedenen Grössen angeboten werden.

Denkbar sind auch Ausführungen die entlang einer Linie 2 zum Anlegen geöffnet und wieder geschlossen werden können und dabei eventuell auch in bezug auf ihren Durchmesser dem jeweiligen Träger oder der jeweiligen Trageposition anpassbar sind. Hierzu kann ein Klettverschluss vorgesehen sein, wobei das eine Ende der Kompressions- und Stützbandage 1 mit einem Haken- und das andere Ende mit einem Flauschband versehen werden kann. Es sind aber auch andere Verschlussarten möglich, bis hin zum Reissverschluss. In normaler Ausführung kann die Kompressions- und Stützbandage 1 entlang der Linie 2 zusammengenäht sein.

Für die Erfindung wesentlich ist, dass die Kompressions- und Stützbandage 1 an ihrer inneren, dem Körper zuzuwendenden Seite mindestens einen rutschhemmenden Bereich 3 aufweist. Vorzugsweise ist dieser rutschhemmende Bereich 3 in Form eines Streifens auf die üblicherweise aus einem Gewebe bestehende Bandagenfläche 4 aufgebracht, wie dies auch aus Fig. 2 ersichtlich ist. Wie in Fig. 3 dargestellt, sind auch zwei oder mehr parallel nebeneinander liegende streifenförmige rutschhemmende Bereiche 3 und 5 möglich. Sinnvollerweise ist bei der schlauchförmigen Kompressions- und Stützbandage 1 mindestens je ein streifenförmiger rutschhemmender Bereich 3 und 6 entlang jeder Längskante 7 und 8 vorgesehen.

Die rutschhemmenden Bereiche 3, 5 und 6 bestehen vorzugsweise aus einem gummielastischem Material, beispielsweise aus Silikon. Dieses kann als dünne Schicht auf das Gewebe der Kompressions- und Stützbandage 1 bzw. 9 aufgebracht werden. Die dünne Schicht wirkt beim Tragen am Körper stark rutschhemmend, wird aber beispielsweise am Bein oder Arm nicht als störend empfunden. Anstelle von Silikon sind selbstverständlich auch andere rutschhemmende Materialien denkbar, sei es mit glatter oder mit aufgerauhter Oberfläche. Die vorgeschlagene Streifenform der rutschhemmenden Bereiche 3, 5 und 6 begünstigt nicht nur die gewünschte Wirkung, sie ist auch bei der Herstellung vorteilhaft. Das zur Kompressions- und Stützbandage 1 oder 9 zu verarbeitende Band kann kontinuierlich durch eine Beschichtungseinrichtung laufen. Das Band kann danach abgeschnitten und als Wickelbandage angeboten oder zur schlauchförmigen Kompressions- und Stützbandage 1 nach Fig. 1 konfektioniert werden.

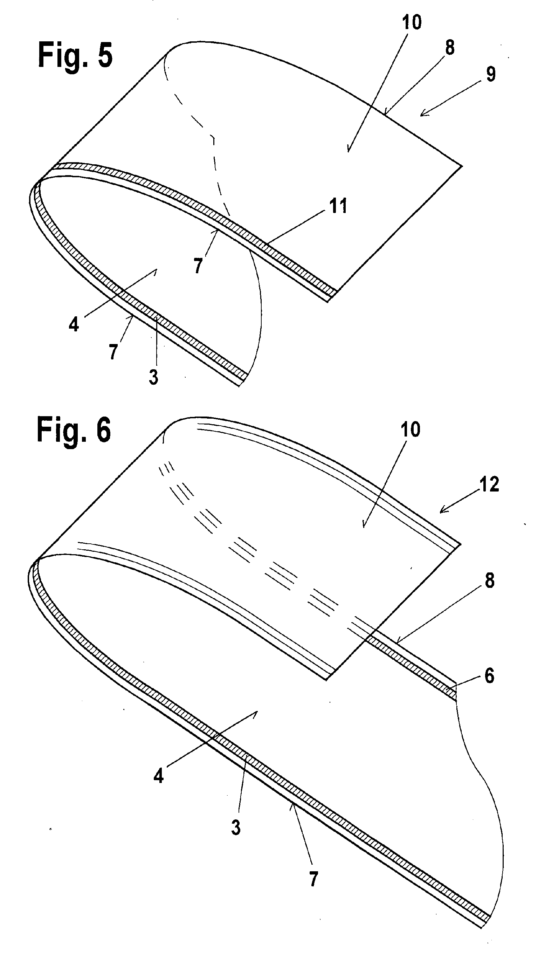

Bei einer bandförmigen, zu wickelnden Kompressions- und Stützbandage 9 kann es nach den Fig. 4 und 5 von Vorteil sein, wenn beide Seiten, d.h. die innere und die äussere Bandagenfläche 4 und 10, mit jeweils mindestens einem rutschhemmenden Bereich 3 und 11 versehen wird. Dadurch wird die Haftung der einzelnen Wickellagen aufeinander so weit verbessert, dass sich diese auch untereinander nicht mehr verschieben können. Der Sinn dieser Ausführung hängt von der Materialbeschaffenheit des Gewebes der Kompressions- und Stützbandage 9 und vom gewählten rutschhemmenden Material ab. Ist die Haftung des rutschhemmenden Bereiches 3 auf dem Gewebe selbst schon gut genug, dann kann auf das rutschsichere Aufeinanderlegen je eines rutschhemmenden Bereiches 3 und 11 verzichtet werden. Möglich ist es auch hier, anstatt wie in Fig. 5 dargestellt, nicht nur entlang einer Längskante 7, sondern auch entlang der gegenüberliegenden Längskante 8 einen streifenförmigen rutschhemmenden Bereich 3 vorzusehen.

In Fig. 6 ist eine Kompressions- und Stützbandage 12 dargestellt, mit nur auf der einen Seite, bzw. Bandagenfläche 4 angebrachten rutschhemmenden Bereichen 3 und 6. Dies jedoch entlang beider Längskanten 7 und 8. Diese Ausführung entspricht weitgehend derjenigen nach Fig. 1, nur dass sie zu wickeln anstatt zum Überziehen ist.

Zur Fixierung einer bandförmigen Kompressions- und Stützbandage 1 können im Bereich von deren einem Bandende 13 zwei Befestigungsmittel 14 und 15 vorgesehen. Diese sind als Klammern ausgebildet und weisen an ihren freien Enden Zacken 16 auf. Letztere sind, wie an sich bekannt, dazu bestimmt, sich in das Gewebe der Kompressions- und Stützbandage einzuhaken. An ihrem diesen Zacken 16 gegenüberliegenden Ende 17 sind die Befestigungsmittel 14 und 15 fest mit dem Bandende 13 verbunden. Dies kann durch Festklemmen erfolgen, wobei die Befestigungsmittel 14 und 15 zwei Schenkel aufweisen, die das Bandende 13 umklammern. Die Schenkel sind vorteilhaft mit Ausstanzungen versehen, die sich im Gewebe festkrallen. Eine weitere Möglichkeit liegt darin, an diesem Ende 17 der Befestigungsmittel 14 und 15 Löcher vorzusehen und sie am Bandende 13 anzunähen. Auch ein Ankleben wäre denkbar. Entscheidend ist lediglich, dass die Befestigungsmittel 14 und 15 unverlierbar mit der Kompressions- und Stützbandage 1 verbunden bleiben.

Im Bereich des die Befestigungsmittel 14 und 15 aufweisenden Bandendes 13 der Kompressions- und Stützbandage 1 ist eine Tasche 18 ausgebildet. Diese kann dadurch gebildet werden, dass das Band umgeschlagen und entlang der aufeinander liegenden Bandkanten mit einer Naht 19 angenäht, bzw. schmalkantig abgesteppt wird. Die den Befestigungsmitteln 14 und 15 abgewendete Seite 20 der Tasche 18 bleibt offen. Diese Seite 20 entspricht dem ursprünglichen Ende des Bandes. Die Befestigungsmittel 14 und 15 sind an der Bruchkante angebracht, die das neue Bandende bildet. Anstelle der Naht 19 ist auch ein Verkleben oder Verschweissen der Bandseiten denkbar.

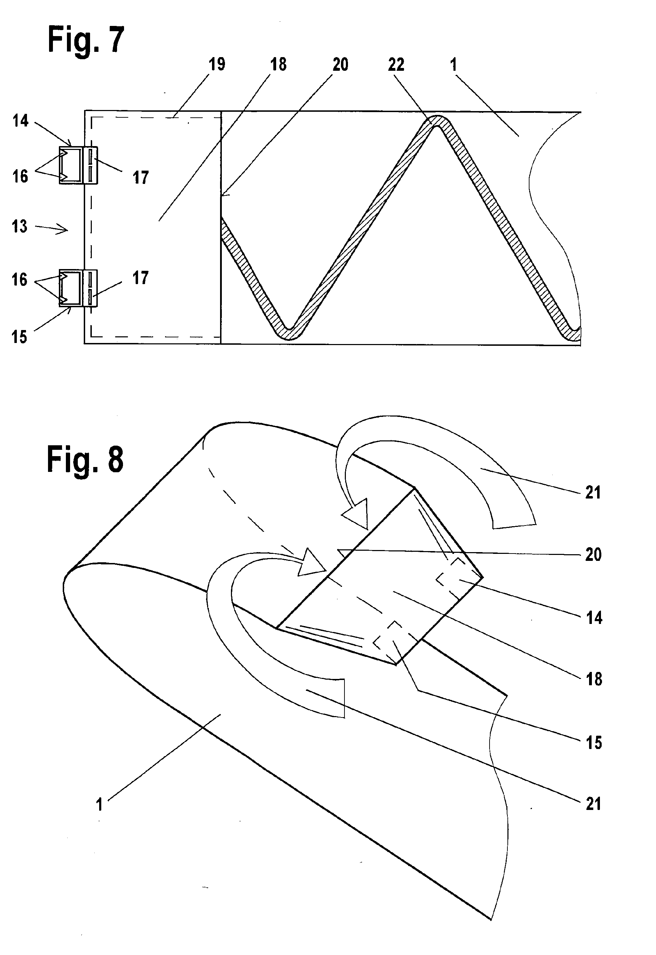

Beim Waschen der Kompressions- und Stützbandage 1 wird deren die Befestigungsmittel 14 und 15 aufweisendes Bandende 13 gemäss den Pfeilen 21 in Fig. 8 so umgestülpt, dass die Befestigungsmittel 14 und 15 in das Innere der Tasche 18 zu liegen kommen. Die Befestigungsmittel 14 und 15 liegen nun nicht mehr frei. Sie sind vollkommen geschützt in der Tasche 18. Das Umstülpen des weichen und gewöhnlich elastischen Bandes geht sehr leicht. Dennoch kann sich die Tasche 18 während des Waschens nicht ungewollt öffnen. Das Waschen in der Waschmaschine bereitet keine Schwierigkeiten mehr.

Die Kompressions- und Stützbandage 1 kann allenfalls auch bei Nichtgebrauch mit umgestülpter Tasche 18 aufbewahrt werden, so dass ein unerwünschtes Verhaken ausgeschlossen ist. In jedem Fall können sich die Befestigungsmittel 14 und 15 nicht lösen und verloren gehen.

Alternativ könnte das Band lediglich nach Fig. 7 umgeschlagen und auf die Naht 19 verzichtet werden. In diesem Fall müsste aber beim Waschen das der Seite 20 entsprechende freie Ende des Bandes um die Befestigungsmittel 14 und 15 gelegt und in dieser Lage befestigt werden, um auf diese Weise eine schützende Aufnahme oder Abdeckung für die Befestigungsmittel 14 und 15 zu bilden. Nachteilig wäre, dass dies eventuell zusätzliche Mittel notwendig machen könnte, um diese Aufnahme oder Abdeckung geschlossen zu halten, z.B. ein Klettverschluss. Die gezeichnete Ausführung der Erfindung ist daher vorzuziehen.

Aus Fig. 7 ist auch eine weitere Variante eines rutschhemmenden Bereiches 22 ersichtlich. Dieser ist hier als sich über die Länge der Kompressions- und Stützbandage 1 erstreckende Schlangenlinie, deren Bögen annähernd von Seitenkante zu Seitenkante des Bandes verlaufen. Die rutschhemmende Wirkung ist besonders gross. Und dies auch dann wenn das Wickeln der Kompressions- und Stützbandage 1 nicht besonders sorgfältig erfolgt.

Es liegt im Rahmen der Erfindung die Kompressions- und Stützbandage auch anders als gezeichnet und vorgehend beschrieben auszubilden. Die beschriebenen Merkmale können beliebig miteinander kombiniert oder in abgewandelter Form verwirklicht werden.

Die erfindungsgemässe Kompressions- und Stützbandage kann beliebig an den Beinen, Armen, am Bauch oder sonst am Körper verwendet werden. Und dies nicht nur beim Menschen, sondern auch bei Tieren.

## Patentansprüche

1. Kompressions- und Stützbandage, **dadurch gekennzeichnet, dass** sie mindestens an ihrer dem Körper zuzuwendenden Bandagenfläche (4) mindestens einen rutschhemmenden Bereich (3, 5, 6, 22) aufweist.

2. Kompressions- und Stützbandage nach Anspruch 1, **dadurch gekennzeichnet, dass** der rutschhemmende Bereich (3, 5, 6, 22) in Form mindestens eines Streifens auf die beispielsweise aus einem Gewebe bestehende Bandagenfläche (4) aufgebracht ist.

3. Kompressions- und Stützbandage nach Anspruch 1 oder 2, **dadurch gekennzeichnet, dass** der rutschhemmende Bereich (3, 5, 6, 22) als Beschichtung auf die Bandagenfläche (4) aufgebracht ist.

4. Kompressions- und Stützbandage nach einem der Ansprüche 1 - 3, **dadurch gekennzeichnet, dass** entlang jeder der beiden Längskanten (7, 8) der Kompressions- und Stützbandage (1, 12) mindestens ein rutschhemmender Bereich (3, 5, 6) angeordnet ist.

5. Kompressions- und Stützbandage nach einem der Ansprüche 1 - 4, **dadurch gekennzeichnet, dass** der rutschhemmende Bereich (22) als sich über mindestens einen Teil der Länge der Kompressions- und Stützbandage (1) erstreckende Schlangenlinie ausgebildet ist, deren Bögen z.B. von Seitenkante zu Seitenkante der Kompressions- und Stützbandage (1) verlaufen.

6. Kompressions- und Stützbandage nach einem der Ansprüche 1 - 5, **dadurch gekennzeichnet, dass** beide Seiten, d.h. sowohl die innere, dem Körper zuzuwendende Bandagenfläche (4), als auch die äussere, vom Körper abzuwendende Bandagenfläche (10) der Kompressionsund Stützbandage (9), mit jeweils mindestens einem rutschhemmenden Bereich (3, 11) versehen ist.

7. Kompressions- und Stützbandage nach einem der Ansprüche 1 - 6, **dadurch gekennzeichnet, dass** der rutschhemmende Bereich (3, 5, 6, 11, 22) aus einem gummielastischen Material besteht.

8. Kompressions- und Stützbandage nach Anspruch 7, **dadurch gekennzeichnet, dass** der rutschhemmende Bereich (3, 5, 6, 11, 22) aus Silikon besteht.

9. Kompressions- und Stützbandage nach einem der Ansprüche 1 - 8, **dadurch gekennzeichnet, dass** der rutschhemmende Bereich (3, 5, 6, 11, 22) eine glatte Oberfläche aufweist.

10. Kompressions- und Stützbandage nach einem der Ansprüche 1 - 8, **dadurch gekennzeichnet, dass** der rutschhemmende Bereich (3, 5, 6, 11, 22) eine aufgerauhte Oberfläche aufweist.

11. Kompressions- und Stützbandage nach einem der Ansprüche 1 - 10, **dadurch gekennzeichnet, dass** die äussere, vom Körper abzuwendende Bandagenfläche (10) der Kompressions- und Stützbandage (9), mit jeweils mindestens einem rutschhemmenden Bereich (11) versehen ist, der auf der jeweils darüber zu liegen kommenden Lage der Bandagenfläche (4) eine rutschhemmende Wirkung hat, beispielsweise indem dieser rutschhemmende Bereich (11) entsprechend einem Klettverschluss entweder nach Art eines Flauschbandes oder nach Art eines Hakenbandes ausgebildet ist.

12. Kompressions- und Stützbandage nach einem der Ansprüche 1 - 11, **gekennzeichnet durch** einen Verschluss zum Verbinden von zwei freien Enden der Kompressions- und Stützbandage (1, 9, 12), z.B. ein Klettverschluss oder ein Reissverschluss.

13. Kompressions- und Stützbandage nach einem der Ansprüche 1 - 12, **gekennzeichnet durch** im Bereich von deren Bandende (13) angeordnete Befestigungsmittel (14, 15), wobei entweder eine Tasche (18) oder eine Aufnahme für diese Befestigungsmittel (14, 15) vorhanden ist, mit dem Zweck, die Befestigungsmittel (14, 15) während des Waschens oder Nichtgebrauchs der Kompressions- und Stützbandage (1) geschützt unterzubringen.

14. Kompressions- und Stützbandage nach Anspruch 13, **dadurch gekennzeichnet, dass** die Tasche (18) oder Aufnahme für die Befestigungsmittel (14, 15) dadurch gebildet ist, dass das Band der Bandage oder des Verbandes (1) umgeschlagen ist.

15. Kompressions- und Stützbandage nach Anspruch 14, **dadurch gekennzeichnet, dass** das Band umgeschlagen und dessen aufeinander liegende Teile teilweise miteinander verbunden sind, derart dass eine Tasche (18) gebildet ist.

16. Kompressions- und Stützbandage nach Anspruch 15, **dadurch gekennzeichnet, dass** die den Befestigungsmitteln (14,15) abgewendete Seite (20) der Tasche (18) offen ist.

17. Kompressions- und Stützbandage nach Anspruch 15 oder 16, **dadurch gekennzeichnet, dass** die aufeinander liegenden Teile oder Bandkanten entweder mit einer Naht (19) angenäht sind oder verklebt oder verschweisst sind.

18. Kompressions- und Stützbandage nach einem der Ansprüche 14 - 17, **dadurch gekennzeichnet, dass** die Befestigungsmittel (14, 15) im Bereich der durch das Umschlagen gebildeten Bruchkante befestigt sind.

19. Kompressions- und Stützbandage nach einem der Ansprüche 1 - 18, **dadurch gekennzeichnet, dass** die Befestigungsmittel (14 , 15) als Klammern ausgebildet sind, die mit ihrem einen Ende am Band der Kompressions- und Stützbandage (1) befestigt sind und an ihrem freien Ende Zacken (5) aufweisen, die dazu bestimmt sind, sich in das Gewebe der Kompressions- und Stützbandage (1) einzuhaken.
